Europäisches Patentamt

European Patent Office

Office européen des brevets

(19)

(11) **EP 0 989 117 B1**

(12) **FASCICULE DE BREVET EUROPEEN**

(45) Date de publication et mention
de la délivrance du brevet:
**24.10.2001 Bulletin 2001/43**

(51) Int Cl.⁷: **C07D 201/04**

(21) Numéro de dépôt: **99402107.9**

(22) Date de dépôt: **24.08.1999**

(54) **Procédé de préparation de lactames à partir des cycloalcanones oximes correspondantes**

Verfahren zur Herstellung von Lactamen ausgehend von den entsprechenden Cycloalcanonoximen

Process for the preparation of lactames from the corresponding cycloalcanone oximes

(84) Etats contractants désignés:
**AT BE CH CY DE DK ES FI FR GB GR IE IT LI LU
MC NL PT SE**

(30) Priorité: **21.09.1998 FR 9811733**

(43) Date de publication de la demande:
**29.03.2000 Bulletin 2000/13**

(73) Titulaire: **Atofina
92800 Puteaux (FR)**

(72) Inventeur: **Ollivier, Jean
64260 Arudy (FR)**

(74) Mandataire: **Neel, Henry et al
Atofina
D.C.R.D./D.P.I.
4, Cours Michelet,
La Défense 10
92091 Paris La Défense Cedex (FR)**

(56) Documents cités:
**FR-A- 2 417 501          US-A- 2 721 199**

**Description**

La présente invention se rapporte à la préparation de lactames qui sont utilisés comme monomères de base des polyamides. Plus précisément, elle concerne un procédé de préparation de lactames à partir des cycloalcanone oximes correspondantes par transposition selon la réaction de Beckmann dans lequel on utilise l'acide méthanesulfonique.

La réaction de transposition selon Beckmann qui consiste à convertir des cétoximes en amides substitués correspondants au moyen de réactifs acides est connue depuis fort longtemps.

Cette réaction est mise à profit pour la production industrielle de lactames à partir de cétoximes cycliques, plus particulièrement pour former le caprolactame et le lauryllactame qui sont les monomères de base des polyamides 6 et 12 respectivement.

Pour effectuer la transposition de Beckmann, on a proposé d'utiliser différents réactifs acides.

On a décrit l'utilisation d'acide sulfurique seul (voir DE-B-15 45 653 et FR-A-2 417 501) ou en mélange avec de l'acide trifluoroacétique (voir JP-A-51034185) ou du trioxyde de soufre et de l'acide chlorosulfonique (voir JP-A-57031660).

On a proposé d'employer l'acide phosphorique (voir CH-A-530402 et JP-A-62149665) ou l'acide polyphosphorique (voir DE-B-1 545 617).

On a aussi décrit l'usage d'acide acétique (voir CH-A-394212), d'un mélange d'acide acétique et d'acide cyanurique (voir JP-B-71023740), d'un mélange d'acide acétique et d'acétone (voir JP-A-51004163), d'un mélange d'acide acétique, d'acétone et d'un catalyseur fluoré (voir JP-A-51004164) et d'un mélange d'acide acétique ou d'anhydride acétique et d'acide fluorhydrique (voir US-A-3 609 142).

Enfin, on a proposé d'utiliser de l'acide chlorhydrique conjointement avec un solvant organique polaire (voir DE-A-1620478) ou avec un catalyseur, par exemple un sel métallique (voir US-A-3 904 608) ou un mélange de silice et d'alumine.

L'acide le plus largement employé sur le plan industriel est de loin l'acide sulfurique. Ce dernier n'est cependant pas exempt d'inconvénients.

Il est connu que, dans les conditions de température de la transposition (supérieure à 135°C), l'acide sulfurique est un facteur favorisant l'apparition de réactions parasites d'hydrolyse. Cette hydrolyse se produit sur la cycloalcanone oxime de départ qu'elle transforme en cétone d'une part, et sur le lactame final qu'elle convertit en acide aminé, d'autre part. Il en résulte une diminution de la production de lactame et des difficultés supplémentaires dans les étapes subséquentes de séparation et de purification du lactame.

Lorsque l'oxime à traiter renferme un solvant chloré résiduel venant de l'étape précédente, comme c'est notamment le cas lorsqu'on procède par photonitrosation de cycloalcane, deux réactions parasites apparaissent.

La première réaction induit une décomposition partielle de l'acide sulfurique avec libération de dioxyde de soufre. Au cours des différentes opérations de recyclage de la phase organique contenant le cycloalcane n'ayant pas réagi, la teneur en dioxyde de soufre augmente ce qui a pour effet de ralentir la réaction de photonitrosation.

La deuxième réaction parasite provoque une décomposition du solvant chloré résiduel en phosgène toxique pour l'homme.

Enfin, tous les effluents contenant de l'acide sulfurique générés par le procédé industriel ne peuvent être recyclés qu'au prix d'un traitement long, difficile et coûteux.

Il a maintenant été trouvé que l'on peut pallier les inconvénients précités, et contribuer ainsi à améliorer la rentabilité de l'installation industrielle, en remplaçant l'acide sulfurique par l'acide méthanesulfonique.

L'invention a donc pour objet un nouveau procédé de préparation de lactames contenant 6 à 12 atomes de carbone à partir des cycloalcanone oximes correspondantes par transposition selon la réaction de Beckmann, ce procédé étant caractérisé en ce que l'acide mis en oeuvre est l'acide méthanesulfonique.

La transposition de Beckmann est généralement mise en oeuvre dans un réacteur opérant à chaud et sous une agitation vigoureuse.

La cycloalcanone oxime est généralement introduite dans le réacteur sous la forme d'une solution contenant 10 à 40 % en poids d'oxime, de préférence 25 à 35 %, dans l'acide méthanesulfonique.

Pour des raisons évidentes de sécurité liées à la très forte exothermicité de la réaction, on préfère introduire la solution d'oxime dans un réacteur qui contient un volume adéquat d'acide méthane sulfonique maintenu à la température requise pour la effectuer la transposition. Ce volume peut, comme le sait l'homme du métier, varier dans une large mesure selon que la réaction est mise en oeuvre de manière continue ou discontinue.

Le titre pondéral de l'acide méthanesulfonique est généralement compris entre 70 et 90 %, de préférence 95 et 99 %.

On procède en général à une température comprise entre 120 et 180°C, de préférence 140 et 160°C, et pendant une durée telle que le temps de séjour dans le réacteur varie de 2 minutes à 1 heure, de préférence 15 à 30 minutes.

La transposition est mise en oeuvre sous une agitation vigoureuse. Dans la présente invention, l'expression "agitation vigoureuse" s'entend d'une agitation présentant un nombre de Reynolds (Re) supérieur à 10000, calculé selon la formule.

$$Re = l^2\, n\, \rho\, /\, \mu$$

dans laquelle

> l est le diamètre de l'élément d'agitation
> n est le nombre de tours par seconde
> ρ est la masse volumique du milieu réactionnel
> μ est la viscosité du milieu réactionnel.

A l'issue de la réaction, on récupère le lactame dans l'acide méthanesulfonique. Cette solution est généralement soumise à une ou plusieurs étapes de séparation et de purification bien connues de l'homme du métier. L'acide méthanesulfonique récupéré peut facilement être purifié, par exemple par simple distillation, afin qu'on puisse le recycler dans le procédé.

Les exemples qui suivent permettent d'illustrer l'invention.

## EXEMPLE 1

A 100 g d'acide méthanesulfonique à 90 % en poids, maintenu à 120°C et sous agitation (Re > 10000), on ajoute en 1 heure 231 g d'une solution contenant 31 % en poids de l'oxime de la cyclododécanone (0,363 mole) dans de l'acide méthanesulfonique. Le milieu réactionnel est porté à 135-140°C pendant 1 heure pour parachever la transposition.

A la fin de la réaction, on récupère 70,9 g de lauryllactame (rendement : 99 %). On ne trouve pas trace d'acide aminé résultant de l'hydrolyse du lauryllactame.

## EXEMPLE 2 (COMPARATIF)

A 100 g d'acide sulfurique à 98 % en poids, maintenu à 120°C et sous agitation (Re > 10000), on ajoute en une heure 250 g d'une solution contenant 30 % en poids d'oxime de la cyclododécanone (0,38 mole) dans l'acide sulfurique.

Après 1 heure de réaction à 135-140°C, on récupère 73,12 g de lauryllactame (rendement : 97,5 %).

Le milieu réactionnel contient en outre 1,125g de cyclododécanone et 0,75 g d'acide amino-12 dodécanoïque.

On produit 1,44 g de dioxyde de soufre. Les gaz de transposition contiennent du phosgène.

## EXEMPLE 3

A 100 g d'acide méthanesulfonique à 90 % en poids, maintenu à 120°C et sous agitation (Re > 10000), on ajoute en 1 heure 225 g d'une solution contenant 35 % en poids de l'oxime de la cyclohexanone (0,697 mole) dans l'acide méthanesulfonique. Le milieu réactionnel est porté à 135-140°C pendant 1 heure pour parachever la transposition.

A la fin de la réaction, on récupère 77,96 g de caprolactame (rendement : 99 %). On ne trouve pas trace d'acide aminé résultant de l'hydrolyse du caprolactame.

## EXEMPLE 4 (COMPARATIF)

A 100 g d'acide sulfurique à 98 % en poids, maintenu à 120°C et sous agitation (Re > 10000), on ajoute en une heure 228 g d'une solution contenant 35 % en poids d'oxime de la cyclohexanone (0,706 mole) dans l'acide sulfurique.

Après 1 heure de réaction à 135-140°C, on récupère 78,2 g de caprolactame (rendement : 98 %).

Le milieu réactionnel contient en outre 1,125g de cyclododécanone et 0,75 g d'acide amino-12 dodécanoïque.

On produit 1,4 g de dioxyde de soufre. Les gaz de transposition contiennent 20 ppm de phosgène.

## Revendications

1. Procédé de préparation de lactames contenant 6 à 12 atomes de carbone à partir des oximes de cycloalcanone correspondantes par transposition selon la réaction de Beckmann en présence d'acide, **caractérisé en ce qu'**on utilise l'acide méthanesulfonique.

2. Procédé selon la revendication 1, **caractérisé en ce que** l'on utilise l'oxime de cycloalcanone sous la forme d'une solution contenant 10 à 40 % en poids d'oxime dans l'acide méthanesulfonique.

3. Procédé selon la revendications 2, **caractérisé en ce que** la solution contient 25 à 35 % en poids d'oxime.

4. Procédé selon l'une des revendications 1 à 3, **caractérisé en ce que** le titre pondéral de l'acide méthanesulfonique est compris entre 70 et 90 %.

5. Procédé selon l'une des revendications 1 à 4, **caractérisé en ce que** l'on opère à une température comprise entre 120 et 180°C.

6. Procédé selon l'une des revendications 1 à 5, **caractérisé en ce que** l'on opère sous une agitation présentant un nombre de Reynolds supérieur à 10000.

## Claims

1. Process for the preparationof lactums comprising 6 to 12 carbon atoms from the corresponding cycloalkanone oximes by rearrangement according to the Beckmann reaction in the presence of acid, **characterized in that** the use is made of methanesulphonic acid.

**2.** Process according to Claim 1, **characterized in that** the cycloalkanone oxime is used in the form of a solution comprising 10 to 40% by weight of oxime in methanesulphonic acid.

**3.** Process according to Claim 2, **characterized in that** the solution comprises 25 to 35% by weight of oxime.

**4.** Process according to one of Claims 1 to 4, **characterized in that** the strength by weight of the methanesulphonic acid is between 70 and 90%.

**5.** Process according to one of Claims 1 to 4, **characterized in that** the reaction is carried out at a temperature of between 120 and 180°C.

**6.** Process according to one of Claims 1 to 5, **characterized in that** the reaction is carried out with stirring which exhibits a Reynolds number of greater than 10,000.

**Patentansprüche**

**1.** Verfahren zur Herstellung von Lactamen, die 6- bis 12-Kohlenstoffatome enthalten, aus den entsprechenden Cycloalkanonoximen durch Beckmann-Umlagerung in Gegenwart einer Säure, **dadurch gekennzeichnet, daß** Methansulfonsäure verwendet wird.

**2.** Verfahren nach Anspruch 1, **dadurch gekennzeichnet, daß** das Cycloalkanonoxim in Form einer Lösung verwendet wird, die 10 bis 40 Gew.-% Oxim in Methansulfonsäure enthält.

**3.** Verfahren nach Anspruch 2, **dadurch gekennzeichnet, daß** die Lösung 25 bis 35 Gew.-% Oxim enthält.

**4.** Verfahren nach einem der Ansprüche 1 bis 3, **dadurch gekennzeichnet, daß** der gewichtsbezogene Gehalt an Methansulfonsäure 70 bis 90 % beträgt.

**5.** Verfahren nach einem der Ansprüche 1 bis 4, **dadurch gekennzeichnet, daß** die Umsetzung bei einer Temperatur von 120 bis 180 °C durchgeführt wird.

**6.** Verfahren nach einem der Ansprüche 1 bis 5, **dadurch gekennzeichnet, daß** das Reaktionsgemisch gerührt wird, wobei die Reynolds-Zahl mehr als 10000 beträgt.